# EUROPEAN PATENT SPECIFICATION

(11) **EP 3 833 390 B1**
(45) Date of publication and mention of the grant of the patent: **01.10.2025**
(21) Application number: 19845946.3
(22) Date of filing: 07.08.2019
(51) Int. Cl.: C07K 16/28, A61K 39/00, A61K 39/395, A61P 35/00, C12N 5/0783, C12N 5/09, A61K 40/11, A61K 40/42, A61K 35/17

(54) **ENHANCED DELIVERY OF DRUGS AND OTHER COMPOUNDS TO THE BRAIN AND OTHER TISSUES**
VERBESSERTE ABGABE VON MEDIKAMENTEN UND ANDEREN VERBINDUNGEN AN DAS GEHIRN UND ANDERES GEWEBE
AMÉLIORATION DE L'ADMINISTRATION DE MÉDICAMENTS ET AUTRES COMPOSÉS AU CERVEAU ET AUTRES TISSUS

(30) Priority: 09.08.2018 US 201862716563 P
(43) Date of publication of application: 16.06.2021
(60) Divisional application: 25165709.4
(73) Proprietor: Duke University, Durham, NC 27705 (US)
(72) Inventor: SAMPSON, John H., Durham, North Carolina 27710 (US); GEDEON, Patrick C., Durham, North Carolina 27713 (US); CHOI, Bryan D., Durham, North Carolina 27705 (US)
(74) Representative: Tombling, Adrian George
(86) International application number: PCT/US2019/045437
(87) International publication number: WO 2020/033499

(56) References cited:
- WO-A1-2013/185010
- US-A1- 2010 129 392
- US-A1- 2015 132 306
- AYGUEN SAHIN ET AL: "Development of third generation anti-EGFRvIII chimeric T cells and EGFRvIII-expressing artificial antigen presenting cells for adoptive cell therapy for glioma", PLOS ONE, vol. 13, no. 7, 5 July 2018 (2018-07-05), pages 1 - 19, XP055684297, DOI: 10.1371/journal.pone.0199414
- PATRICK C. GEDEON ET AL: "A Rationally Designed Fully Human EGFRvIII:CD3-Targeted Bispecific Antibody Redirects Human T Cells to Treat Patient-derived Intracerebral Malignant Glioma", CLINICAL CANCER RESEARCH, vol. 24, no. 15, 27 April 2018 (2018-04-27), US, pages 3611 - 3631, XP055684287, ISSN: 1078-0432, DOI: 10.1158/1078-0432.CCR-17-0126
- PATRICK C GEDEON ET AL: "An EGFRvIII-targeted bispecific T-cell engager overcomes limitations of the standard of care for glioblastoma", EXPERT REVIEW OF CLINICAL PHARMACOLOGY 20141101 EXPERT REVIEWS LTD. GBR, vol. 6, no. 4, 1 July 2013 (2013-07-01), UK, pages 375 - 386, XP055684290, ISSN: 1751-2433, DOI: 10.1586/17512433.2013.811806
- COLE CAROLINE ET AL: "Tumor-targeted, systemic delivery of therapeutic viral vectors using hitchhiking on antigen-specific T cells", NATURE MEDICINE, NATURE PUBLISHING GROUP US, NEW YORK, vol. 11, no. 10, 1 October 2005 (2005-10-01), pages 1073 - 1081, XP002470982, ISSN: 1078-8956, DOI: 10.1038/NM1297
- SINGH KIRIT ET AL: "EXTH-82. T CELL HITCHHIKING AS A MECHANISM OF DRUG DELIVERY TO THE BRAIN", vol. 23, no. Supplement_6, 12 November 2021 (2021-11-12), US, pages vi182 - vi182, XP055901587, ISSN: 1522-8517, Retrieved from the Internet <URL:https://academic.oup.com/neuro-oncology/article-pdf/23/Supplement_6/vi182/41141260/noab196.721.pdf> [retrieved on 20220314], DOI: 10.1093/neuonc/noab196.721
- SCHALLER TEILO H ET AL: "Pharmacokinetic Analysis of a Novel Human EGFRvIII:CD3 Bispecific Antibody in Plasma and Whole Blood Using a High-Resolution Targeted Mass Spectrometry Approach.", JOURNAL OF PROTEOME RESEARCH 02 08 2019, vol. 18, no. 8, 2 August 2019 (2019-08-02), pages 3032 - 3041, XP009534154, ISSN: 1535-3907
- STROHL ET AL: "Bispecific T-Cell Redirection versus Chimeric Antigen Receptor (CAR)-T Cells as Approaches to Kill Cancer Cells", vol. 8, no. 3, 1 January 2019 (2019-01-01), pages 41, XP009516302, ISSN: 2073-4468, Retrieved from the Internet <URL:https://www.ncbi.nlm.nih.gov/pmc/articles/doi/10.3390/antib8030041> [retrieved on 20190703], DOI: 10.3390/ANTIB8030041
- GEDEON PATRICK C ET AL: "The effect of adoptive transfer of ex vivo activated T cells on the efficacy and tumor penetrance of intravenously-administered CD3-engaging bispecific antibody.", JOURNAL OF CLINICAL ONCOLOGY, vol. 37, no. 8_suppl, 10 March 2019 (2019-03-10), pages 30 - 30, XP093173904, ISSN: 0732-183X, Retrieved from the Internet <URL:https://dx.doi.org/10.1200/JCO.2019.37.8_suppl.30> DOI: 10.1200/JCO.2019.37.8_suppl.30
- GEDEON, P. C. ET AL.: "A rationally designed fully human EGFRvIII: CD 3-targeted bispecific antibody redirects human T cells to treat patient- derived intracerebral malignant glioma", CLINICAL CANCER RESEARCH, vol. 24, no. 15, 27 April 2018 (2018-04-27), pages 3611 - 3631, XP055684287
- GEDEON, P. C. ET AL.: "An EGFRvIII-targeted bispecific T- cell engager overcomes limitations of the standard of care for glioblastoma", EXPERT REVIEW OF CLINICAL PHARMACOLOGY, vol. 6, no. 4, 2013, pages 375 - 386, XP055684290
- SAHIN, A. ET AL.: "Development of third generation anti-EGFRvIII chimeric T cells and EGFRvIII-expressing artificial antigen presenting cells for adoptive cell therapy for glioma", PLOS ONE, vol. 13, no. 7, 5 July 2018 (2018-07-05), pages 1 - 19, XP055684297

## Description

This invention was made with government support under grant numbers R01NS085412, U01NS090284, and F30CA196199 awarded by the U.S. National Cancer Institute. The U.S. government has certain rights in the invention.

### TECHNICAL FIELD OF THE INVENTION

This disclosure relates to the area of drug and other agent delivery to mammalian bodies.

In particular, it relates to increasing penetrance of a drug or other agent to a desired location in the body.

### BACKGROUND OF THE INVENTION

For many drugs and diagnostic agents, no readily available method permits effective access to certain privileged portions of mammalian bodies, such as the CNS. There is a continuing need in the art for methods and products to enhance the localization of molecules to specific tissue types and compartments, including across the blood-brain barrier into the CNS parenchyma.

### SUMMARY OF THE INVENTION

According to the invention there is provided a human bispecific single chain variable fragment (scFv) that binds to EGFRvIII on the surface of tumor cells and to human CD3 on the surface of T cells, and ex vivo-activated T cells for use in treating a glioma in a brain of an immunocompetent human, wherein the T cells are a population of activated antigen-independent T cells.

Further disclosed for context is a method for increasing the amount of an agent to reach a location in a mammalian body. A modified form of the agent is administered to a mammalian body. And modified T cells are administered to the mammalian body. The agent is modified, forming the modified agent, so that it binds to the T cells. The T cells are modified so that they migrate to the location in the mammalian body.

Further disclosed for context is a method for increasing the amount of an EGFRvIII single chain variable fragment to reach a CNS location in a human body. A human EGFRvIII-CD3 bispecific single chain variable fragment (scFv) is administered to a human body. And *ex vivo* activated T cells are administered to the human body.

Further disclosed for context is a method for increasing the amount of an agent to reach a location in a mammalian body. An immunomodulator is administered to the mammalian body whereby T cells migrate to the location in the mammalian body. A modified form of the agent is administered to the mammalian body. The agent is modified, forming the modified agent, so that it binds to the T cells.

Further disclosed for context is a kit for targeting a therapeutic or diagnostic antibody to a location in a mammalian body. An immunomodulator which stimulates T cells to migrate to the location in the mammalian body is administered to the mammalian body. A bifunctional molecule for binding to the T cells and to the therapeutic or diagnostic antibody is administered to the mammalian body. The bifunctional molecule comprises an antibody, antibody fragments, or antibody fragment construct that specifically binds to a T cell surface antigen; and an antibody binding entity selected from the group consisting of: an anti-Fab, an anti-Fc, protein A, and protein L.

Further disclosed for context is a bifunctional molecule for binding to T cells and to a therapeutic or diagnostic antibody. The bifunctional molecule comprises an antibody, antibody fragments, or antibody fragment construct that specifically binds to a T cell surface antigen; and an antibody binding entity selected from the group consisting of: an anti-Fab, an anti-Fc, protein A, and protein L.

Further disclosed for context is a kit for targeting a therapeutic or diagnostic protein or peptide to a location in a mammalian body. The kit comprises an immunomodulator which stimulates T cells to migrate to the location in the mammalian body and a bifunctional molecule for binding to the T cells and to the therapeutic or diagnostic protein or peptide. The bifunctional molecule comprises an antibody, antibody fragments, or antibody fragment construct that specifically binds to a T cell surface antigen and a chemical coupling agent for coupling to the therapeutic or diagnostic protein or peptide.

Further disclosed for context is a bifunctional molecule for binding to the T cells and to the therapeutic or diagnostic protein or peptide. The bifunctional molecule comprises an antibody, antibody fragments, or antibody fragment construct that specifically binds to a T cell surface antigen and a chemical coupling agent for coupling to the therapeutic or diagnostic protein or peptide.

Further disclosed for context is a kit for targeting a therapeutic or diagnostic antibody to a location in a mammalian body. The kit comprises an immunomodulator which stimulates T cells to migrate to the location in the mammalian body and a vector for transforming a T cell to express on its surface an antibody binding entity selected from the group consisting of: an anti-Fab, an anti-Fc, protein A, and protein L.

Further disclosed for context is a method for targeting a therapeutic or diagnostic antibody to a location in a mammalian body. A T cell is transformed to express on its surface an antibody-binding entity selected from the group consisting of: an anti-Fab, an anti-Fc, protein A, and protein L, to form a transformed T cell. The transformed T cell is administered to a mammalian body. The T cell is stimulated *ex vivo* or in the mammalian body to migrate to the location in the mammalian body. A therapeutic or diagnostic antibody is administered to the mammalian body.

Further disclosed for context is a vector for transforming a T cell to express on its surface an antibody binding entity selected from the group consisting of: an anti-Fab, an anti-Fc, protein A, and protein L. The vector comprises a promoter upstream of a coding sequence for the antibody binding entity, wherein the coding sequence comprises a signal sequence, a transmembrane domain, and an extracytoplasmic domain.

These and other examples which will be apparent to those of skill in the art upon reading the specification provide the art with methods and tools for more effectively accessing areas of the mammalian body with therapeutic or diagnostic agents.

### BRIEF DESCRIPTION OF THE DRAWINGS

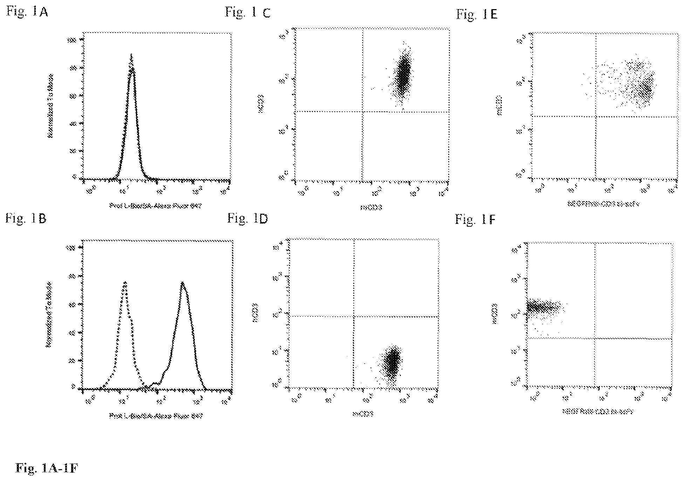

Fig. 1A-1F: Bispecific antibody (hEGFRvIII-CD3 bi-scFv) binds to EGFRvIII receptors on the surface of tumor cells and human CD3 receptors on the surface of T cells. Fig. 1A shows binding of hEGFRvIII-CD3 bis-scFv to CT-2A murine glioma cells; Fig. 1B shows binding of hEGFRvIII-CD3 bis-scFv to CT-2A-EGFRvIII murine glioma cells, Fig. 1C shows human CD3 transgenic splenocytes stained with human CD3 and mouse CD3, Fig. 1D shows wild-type, non-transgenic splenocytes stained with human CD3 and mouse CD3, Fig. 1E shows human CD3 transgenic splenocytes stained with mouse CD3 and hEGFRvIII-CD3 bi-scFv, Fig. 1F shows wild-type, non-transgenic splenocytes stained with mouse CD3 and hEGFRvIII-CD3 bi-scFv.
Fig. 2: EGFRvIII and CD3 binding bispecific antibody (hEGFRvIII-CD3 bi-scFv) redirects human CD3 transgenic murine lymphocytes to induce cytotoxicity of EGFRvIII-positive murine glioma.

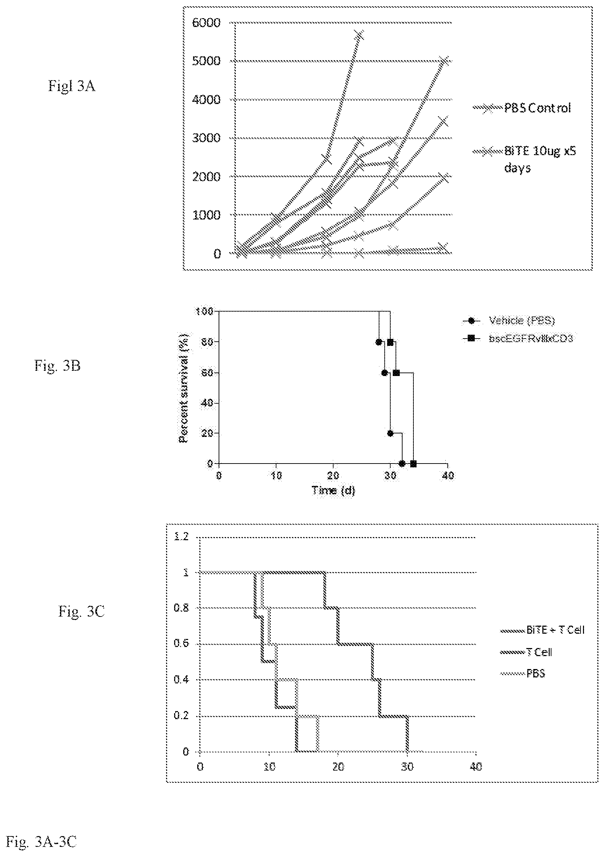

Fig. 3 A-3C: Studies of EGFRvIII-CD3 bispecific antibody (bscEGFRvIIIxCD3) treatment and the effect of adoptive transfer of ex vivo activated T cells in a human CD3 transgenic model with syngeneic B16-EGFRvIII melanoma cells implanted subcutaneously or intracerebrally. Fig. 3A shows subcutaneous tumors effectively treated with bscEGFRvIIIxCD3 antibody in the brain. Fig. 3B shows a modest increase in efficacy observed with hscEGFRvIII-CD3 antibody for tumors implanted in the brain. Fig. 3C shows bsc-EGFRvIII-CD3 antibody significantly extends survival when given in conjunction with ex vivo activated T cells, compared to administration of ex vivo activated T cells alone or vehicle alone.
Fig. 4: Hematoxylin and eosin (H&E) staining of a cross-section of CT-2A-EGFRvIII murine glioma cells six days post orthotopic implantation reveals a highly invasive tumor infiltrating the brain parenchyma.
Fig. 5: IV administration of hEGFRvIII-CD3 bi-scFv effectively treats well-established subcutaneous tumors.
Fig. 6: Administration of *ex vivo* activated T cells enhances EGFRvIII and CD3 binding bispecific antibody (hEGFRvIII-CD3 bi-scFv) efficacy against syngeneic, highly-invasive, orthotopic glioma.
Fig. 7: Following intravenous administration, *ex vivo* activated T cells track to highly invasive, syngeneic, orthotopic glioma.
Fig. 8: Blocking T cell extravasation abrogates the increase in bispecific antibody (hEGFRvIII-CD3 bi-scFv) efficacy obtained with pre- administration of *ex vivo* activated T cells.
Fig. 9: PET/CT imaging demonstrates that pre-administration of *ex vivo* activated T cells increases the biodistribution of intravenously administered CD3 binding bispecific antibody to the brain tumor parenchyma.
Fig. 10: Mass spectroscopy demonstrates that pre-administration of *ex vivo* activated T cells increases the biodistribution of intravenously administered CD3 binding bispecific antibody to the brain parenchyma.

### DETAILED DESCRIPTION OF THE INVENTION

The technical disclosure set out below may in some respects go beyond the scope of the invention, which is defined by the appended claims.

Elements and aspects of the disclosure which do not fall within the scope of the claims are provided for information only, namely to place the actual invention claimed in a broader technical context.

The inventors have developed methods and products to overcome the limitations of certain agents accessing particular compartments of the mammalian body. Being able to target an agent to a particular compartment, whether to enhance or to permit access previously denied, can make effective certain agents that previously were not. It can permit dosing at lower levels to avoid or minimize toxic side effects. The technique is imagined as a kind of molecular hitchhiking, in which an agent hitches a ride on a T cell. The T cell is activated or targeted to traffic to a desired compartment. The T cell is activated *ex vivo* and may be adoptively transferred. The agent is a human bispecific scFv that binds to EGFRvIII on the surface of tumor cells and to human CD3 on the surface of T cells.

T cells may be modified to express antibodies, antibody fragments or antibody fragment constructs. Any fragments that are used must include at least a transmembrane region and an extracellular domain. Different forms of antibodies which may be useful, either as expressed by the T cells, as modified agents, or as linker molecules include bispecific antibodies, single-chain fragment variable (scFv), bispecific diabodies, single-chain bispecific diabodies, bispecific tandem diabodies, single-chain bispecific tandem variable domains; dock-and-lock trivalent Fab, single-domain antibodies, bispecific single-domain antibodies, (Fab')₂, Fab, monovalent IgG, tandem bispecific single chain Fragment variable (bsscFv); single chain triplebody (sctb); two-chain diabody; tandem diabody (TandAb); bispecific Tribody (bsTb); bispecific Bibody (bsBb); dual-affinity re-targeting molecule (DART); mini-ab; immunoligand, etc. Useful antibodies expressed on T cells may include without limitation those that bind to P-selectin, E-selectin, ICAM, VCAM, GlyCAM-1, CD34, and PECAM-1. For any particular tissue to be targeted, an antibody may be used that specifically binds to a protein whose expression in that tissue is enhanced. See The Human Protein Atlas (at the website of the organization Protein Atlas) which provides lists of tissue enriched protein for tissues such as parathyroid gland, placenta, small intestine, kidney, skeletal muscle, duodenum, spleen, epididymis, bone marrow, lymph node, adrenal gland, esophagus, thyroid gland, heart muscle, appendix, tonsil, lung, prostate, rectum, adipose tissue, colon, stomach, uterine cervix, gall bladder, seminal vesicle, breast, ovary, endometrium smooth muscle, salivary gland, pancreas, and urinary bladder.

An immunomodulator may be used to stimulate the T cells to migrate to the location in the mammalian body. The T cells may be stimulated to increase their migration capability or to preferentially migrate to the desired location in the mammalian body. Suitable immunomodulators that may be used include but are not limited to OKT3, a chemokine, an integrin, or combinations of these. Additionally, immunomodulators such as C5a, IL-8, LTB4, kallikrein, and platelet activating factor may be used. Use of such immunomodulators may cause T cells to accumulate in the location. The T cells are a population of activated antigen-independent T cells. Polyclonal activation can be achieved by contacting T cells with phytohaemagglutinin (PHA) or concanavalin A (Con A), for example. Antigen-specific T cells are not required. In some cases it may be useful to deliver the immunomodulator to the location in the mammalian body which one wants to treat. If the immunomodulator is a chemoattractant, for example, it would be suitable to administer the chemoattractant to the location so that the T cells would be stimulated to migrate toward the chemoattractant. The chemoattractant may be administered in a depot form, such that it provides sustained and long-lasting release of chemoattractant.

The modified agent and the modified T cells may be admixed *ex vivo.* Alternatively, they may be separately administered to the mammalian body, preferably within 1 month of each other, within 2 weeks, within 1 week, within 6, 5, 4, 3, 2, or 1 days, or within 18, 12, 6, 5, 4, 3, 2, or 1 hours. In such cases, we understand that the two modified agents bind to each other *in vivo.*

Mammalian bodies which may be subjected to the methods of the invention are human. The bodies may be adult or pediatric.

Vectors may be used for transforming or transfecting T cells to express on their surface molecules not normally expressed by them on their surface. The vector may be a viral vector or a plasmid-based vector, for example. The vector can be used to provide to the T cells the ability to bind, associate, or link to an antibody, such as a diagnostic or therapeutic antibody. Expression by the T cell of the vector construct metaphorically opens the arms of the T cell to hitchhikers that are antibody molecules. The vector comprises a promoter upstream of a coding sequence for an antibody-binding entity. The antibody binding entity is an anti-Fab, and anti-Fc, protein A, protein L, or other specific antibody binding entity. The coding sequence comprises a signal sequence, a transmembrane domain, and an extracytoplasmic domain. The vector may be provided alone or in a kit, for example, with an immunomodulator.

To use such a vector one can transform a T cell. The transformed T cell can be administered (adoptively transferred) to a human body to be treated. Additionally, a therapeutic or diagnostic antibody is administered to the mammalian body, either preincubated with the T-cells or separately administered to the mammalian body. Although applicant does not wish to be bound by any theory or mechanism of action, the antibody will be bound to the T cell by its cell surface-expressed antibody-binding entity.

In some examples we modify T cells, changing their phenotype so that they migrate to specific tissues, organs, or compartments in the body, including across the blood-brain barrier into the CNS parenchyma. The T cells can be modified to alter their tissue localization and/or accumulation and/or migration, either *ex vivo* or *in vivo.* We also modify antibodies and other therapeutic, diagnostic or otherwise useful molecules so that they are able to bind to, become internalized by, or otherwise associate with T cells. In combination, we are able to enhance drug or other agent delivery to specific tissues, organs, or compartments, including to the CNS, by administering the modified T cells and the modified antibodies.

Multiple strategies may be employed to modulate T cell tissue migration. The strategy may include activating T cells, inducing the expression of genes that are known to modulate T cell migration, altering the expression of cell surface receptors, and otherwise modifying T cells such that tissue-specific migration and/or accumulation can be achieved. These modified T cells then enhance the biodistribution of the T cell-associated molecule to specific tissues to which the T cells home. As described below in the examples, using this strategy, CD3-binding bispecific antibodies can be delivered in significantly increased quantities to the CNS parenchyma, producing significant enhancement of therapeutic efficacy.

These data presented here have broad implications for the management of a wide variety of neurologic and non-neurologic conditions, including for example oncologic, infectious, and genetic processes. By modifying T cells, either *ex vivo* or *in vivo,* to traffic to specific tissues through a process that activates them, imparts new receptor specificity, or otherwise modifies T cell trafficking, one can modify the biodistribution of molecules designed to bind to components of T cells. For example, many currently investigated therapeutics including targeted therapies for Alzheimer's disease, Parkinson disease, and other neurodegenerative disease have faced limitations due to poor CNS penetrance following intravenous administration. Similar limitations have been faced with therapeutics, diagnostics and otherwise useful interventions for many non-neurologic disease processes. The methods disclosed here, however, provide a straightforward approach to overcome these limitations.

The above disclosure generally describes the present teachings. The invention is defined by the appended claims.

### Examples

### EXAMPLE 1

### EGFRvIII-CD3-binding bispecific antibody

As a model for our studies, we have used a EGFRvIII and CD3 binding bispecific antibody, hEGFRvIII-CD3 bi-scFv, that we have recently developed and published in *Clinical Cancer Research.*⁴ This therapeutic antibody binds to the tumor specific mutation of the epidermal growth factor receptor, EGFRvIII, as well as to human CD3 on the surface of T lymphocytes (**Figure 1**).

The invasive murine glioma lines CT-2A and CT-2A-EGFRvIII were grown using standard cell culture techniques, harvested at mid-log phase using enzyme free dissociation buffer, washed and incubated with hEGFRvIII-CD3 bi-scFv for 30 minutes. After an additional wash step, a biotinylated Protein L/streptavidin-Alexa Fluor 647 tetramer was used to detect hEGFRvIII-CD3 bi-scFv on the surface of tumor cells. No increase over background was detected in CT-2A samples indicating no bispecific antibody binding (A) while a significant shift in mean fluorescence intensity (MFI) was detected in CT-2A-EGFRvIII samples indicating significant bispecific antibody binding (B). Human CD3 transgenic splenocytes (C and E) or wild-type, non-transgenic splenocytes (D and F) were stained with human CD3 and mouse CD3 (C and D) or mouse CD3 and hEGFRvIII-CD3 bi-scFv (E and F). The human CD3 transgenic splenocytes express both mouse and human CD3 (C) while wild-type, non-transgenic splenocytes express only murine CD3 (D). The presence of human CD3 is necessary for bispecific antibody binding (E and F).

### EXAMPLE 2

### T cell-mediated tumor cell lysis

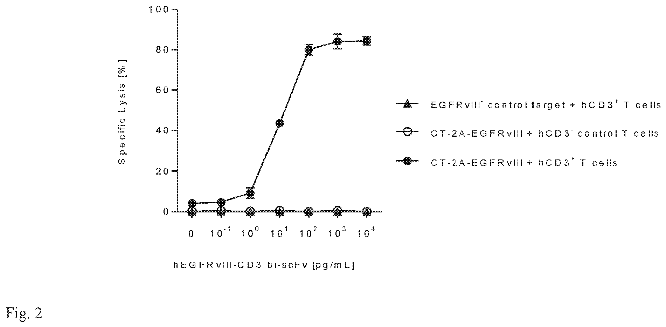

EGFRvIII is a frequent and consistent tumor-specific mutation found on the surface of malignant glioma and other tumors.⁵ This produces a highly immunogenic, cell-surface, tumor-specific epitope⁶ amenable to antibody-based therapy. Tumor cell lysis occurs when the antibody simultaneously binds to the surface of tumor cells and T cells via the EGFRvIII and CD3 receptors, respectively (**Figure 2**).

Chromium-51 release assay was used to assess for cytotoxicity. Note that both target antigen (EGFRvIII) and human CD3 (hCD3) expressing T cells need to be present to mediate cytotoxicity. No cytotoxicity is observed in cases where T cells lacking the human CD3 receptor (hCD3⁻) or target cells lacking the tumor antigen (EGFRvIII⁻) are used.

### EXAMPLE 3

### EGFRvIII-CD3 binding bispecific antibody in a syngeneic B16-EGFRvIII melanoma bearing transgenic mice

When challenged with B16-EGFRvIII melanoma cells subcutaneously, we found the EGFRvIII-CD3 binding bispecific antibody significantly reduces the rate of tumor growth. When B16-EGFRvIII tumors were implanted intracerebrally, however, only a modest increase in survival was induced with EGFRvIII-CD3 binding bispecific antibody compared to vehicle alone. In separate experiments, on the other hand, when ex vivo activated T cells were given in addition to EGFRvIII-CD3 binding bispecific antibody, a significant increase in survival was observed compared to either administration of T cells alone or vehicle alone **(****Figure** 3). Together, these experiments suggested to us that it may be possible that ex vivo modified T cells can be used to modify antibody biodistribution and therefore associated efficacy.

### EXAMPLE 4

### Glioma model with highly invasive cells that infiltrate the surrounding parenchyma and that are thought to reside behind an intact blood-brain barrier

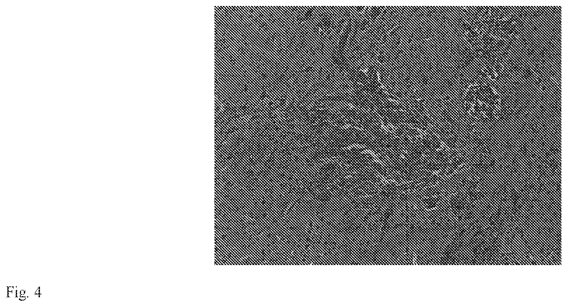

To evaluate for antibody efficacy, we developed a highly aggressive and infiltrative cancer model that replicates the invasive nature of human glioblastoma.⁴ Using this syngeneic, fully-immunocompetent murine glioma model, we were able to test for efficacy against highly invasive tumors that infiltrate the surrounding brain parenchyma **(****Figure 4****).** The invasiveness and syngeneic nature of this model allows us to assess for antibody penetrance in the context of tumor cells that are thought to reside behind an intact blood-brain barrier.

Specifically, given that the CD3 binding portion of EGFRvIII-CD3 binding bispecific antibody binds only to human CD3, we utilized a human CD3 transgenic mouse model (tgε600) with T cells that express functional human CD3 receptors.⁴ The highly aggressive and infiltrative chemically induced murine glioma model, CT-2A-EGFRvIII, is syngeneic in the human CD3 transgenic C57/Bl6 background^{7,8} and infiltrates throughout the brain, recapitulating the invasive nature of human glioblastoma.

To assess tumor growth characteristics, invasiveness and parenchymal infiltration, human CD3 transgenic mice were implanted orthotopically with CT-2A-EGFRvIII glioma. Tumors were allowed to establish for six days (1/3 median untreated survival). On day six post tumor implant, mice were humanly sacrificed to allow for tumor histopathology. A representative H&E section (10x magnification) shows above demonstrates highly invasive syngeneic tumor cells infiltrating the brain parenchyma.

### EXAMPLE 5 (REFERENCE)

### Intravenous administration of EGFRvIII-CD3 binding bispecific antibody cures highly invasive, subcutaneous tumors

Using the model described above, we first assessed antibody efficacy against CT-2A-EGFRvIII tumors implanted in peripheral subcutaneous tissue. While this highly aggressive tumor infiltrates throughout surrounding subcutaneous tissue, peripheral tissue lacks a blood-brain barrier and other factors that restrict antibody penetrance to the CNS. This, therefore, provides a model where high-levels of antibody can accumulate within the tumor following intravenous administration.

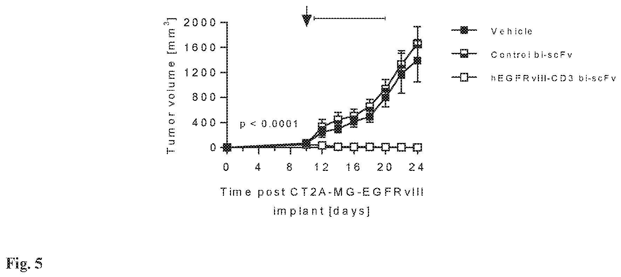

To assess efficacy in this setting, groups of heterozygous human CD3 transgenic mice (n = 10) were implanted subcutaneously with CT-2A-EGFRvIII cells. Tumors were allowed to establish for 10 days following which groups began receiving daily IV treatments with either hEGFRvIII-CD3 bi-scFv, control bi-scFv or an equal volume of vehicle. The difference in tumor growth over time was assessed and a significant difference was observed in the hEGFRvIII-CD3 bi- scFv treated group compared to all other groups (p < 0.0001), with eight out of 10 mice in the hEGFRvIII-CD3 bi-scFv treated group having no detectable tumor burden at the end of the study, while mice from all other groups had reached humane endpoints **(****Figure 5****).** Therefore, in the subcutaneous setting, intravenous administration of EGFRvIII-CD3 binding antibody alone produced sufficient antibody-tumor penetrance to produce highly-effective anti-tumor immune responses.

Cohorts of human CD3 transgenic mice were implanted subcutaneously with CT-2A-EGFRvIII. Tumors were allowed to establish for a period of 10 days. Mice with measurable tumors were then randomized into three groups using a random number generator. Fifty micrograms of hEGFRvIII-CD3 bi-scFv, 50 µg of control bi-scFv or an equivalent volume of vehicle was administered daily for 10 days. Tumor volume was measured every two days. In the hEGFRvIII-CD3 bi-scFv treated group, the mean tumor volume decreases over time post initiation of treatment while in the control and vehicle treated groups tumor volume continues to increase until human endpoints are reached.

### EXAMPLE 6

### Pre-administration with ex vivo activated T cells enhances the efficacy of intravenously administered EGFRvIII-CD3 binding bispecific antibody for the treatment of highly invasive, intracerebral tumors

Having obtained significant efficacy in a subcutaneous *in vivo* tumor model, we next sought to assess for antibody efficacy against orthotopic CT-2A-EGFRvIII glioma. The CNS penetrance of intravenously administered antibodies is severely limited, however, due to the blood-brain barrier and other CNS specific factors. CT-2A-EGFRvIII was implanted orthotopically and cohorts of mice were treated with daily IV injections of hEGFRvIII-CD3 bi-scFv (50 µg, approximately 1.6 mg/kg) or an equivalent dose of control bi-scFv. We found only a modest increase in survival associated with hEGFRvIII-CD3 bi-scFv treatment alone in the orthotopic model (data not shown). Having effectively treated subcutaneous tumors with hEGFRvIII-CD3 bi-scFv alone in an otherwise identical subcutaneous model, we wondered if the differences observed might be due to differences in amounts of hEGFRvIII-CD3 bi-scFv reaching the tumor site in the brain. We hypothesized that activated T cells may enhance efficacy in the orthotopic setting by virtue of trafficking to the tumor site and carrying hEGFRvIII-CD3 bi-scFv molecules loaded on their surface.

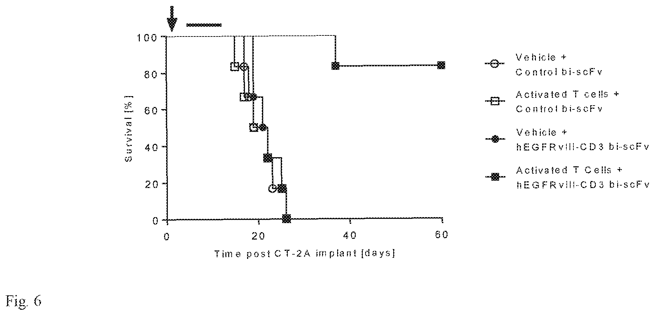

To test this hypothesis, we generated activated T cells from splenocytes harvested from human CD3 transgenic mice. Cells were cultured for 5 days in RPMI-1640 media supplemented with 10% fetal bovine serum (FBS), 2 µg/mL concanavalin A, and 50 IU/mL of IL-2. This culturing protocol was designed to non-specifically expand and activate T cells and does not specifically expand EGFRvIII-specific T cells. Again, we tested for efficacy, including this time groups that received adoptive transfer of activated T cells. We found that pre-administration of activated T cells significantly increased hEGFRvIII-CD3 bi-scFv efficacy, repeatedly producing long-term survivors **(****Figure 6****).**

The highly invasive murine glioma CT-2A-EGFRvIII was implanted orthotopically in human CD3 transgenic mice (females, 8-10 weeks old). Where indicated, on the day of tumor implantation groups of mice (n=10) were given via tail vein injection either vehicle or adoptive transfer of 1x10⁷ *ex vivo* activated T cells derived from human CD3 transgenic mice. The T cells were activated with five days of *in vitro* cell culture stimulation with interleukin-2 and concanavalin A. Where indicated (bar), mice were given 50 µg (approximately 1.6 mg/kg) of hEGFRvIII-CD3 bi-scFv or 50 µg of control bi-scFv by IV injection. Note that a significant increase in efficacy is observed with pre-administration of *ex vivo* activated T cells.

While the above detailed *ex vivo* activation protocol is specific for murine lymphocytes, we have also in separate studies developed and implemented an *ex vivo* lymphocyte activation protocol that can be used clinically for the *ex vivo* activation of human lymphocytes. Briefly, rapidly thawed PBMCs are washed twice in AIM V medium CTS (Thermo Fisher) containing 5% Human AB Serum (vol/vol) (Valley Biomedical), assessed for viability and density (trypan blue) and resuspended at 1 x 10⁶ viable cells per ml in AIM V growth media containing 5% (vol/vol) human AB serum, 300 IU/mL interleukin (IL)-2 (Prometheus Laboratories), and 50 ng / mL OKT3 (Miltenyi Biotech). Cells are grown horizontally in a T150 cell culture flask at 37 °C in a 5% CO2 incubator. After two days of cell culture, cells were harvested and reseeded in growth media without OKT3 at a concentration of 1 x 10⁶ viable cells per ml. When a sufficient number of cells were obtained, after 10-14 days of cell culture, cells are washed twice in PBS and formulated for subsequent intravenous administration. We have used this protocol to generate large numbers of *ex vivo* activated human T cells for clinical use.

### EXAMPLE 7

### Ex vivo activated T cells traffic to the brain parenchyma following intravenous administration

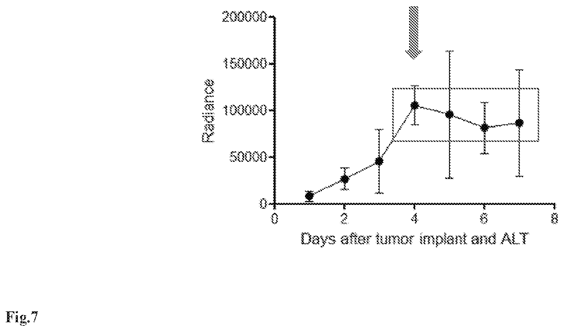

To evaluate further the CNS hitchhiking mechanism of drug delivery, we conducted experiments that allowed us to track *ex vivo* modified T cells following intravenous administration. We isolated splenocytes and modified them *ex vivo* as described above but additionally virally transduced them to express firefly luciferase. This allowed us to perform whole body bioluminescent studies that following administration of luciferin allow for radiance-based quantification of T cell migration. Indeed, we found that intravenously administered *ex vivo* activated T cells migrated from the vascular system to infiltrative tumors located in the brain. The T cells accumulate there, peaking in numbers on average four days post intravenous administration **(****Figure 7****).**

Cohorts of mice with well-established, EGFRvIII-positive, highly-invasive, orthotopic glioma (CT-2A-EGFRvIII) were administered 1x10⁷ *ex vivo* activated T cells that were virally transduced to express firefly luciferase. Each day post tumor implant and adoptive transfer of activated T cells, mice were administered luciferin and whole body bioluminescent imaging allowed for radiance-based quantification of T cells migrating to the intracerebral tumor. T cell accumulation in the intracerebral tumor peaked on average four days (arrow) post adoptive transfer. This T cell migration to the CNS can be exploited to transport large T cell binding macromolecules to the CNS parenchyma.

### EXAMPLE 8

### Blocking T cell extravasation abrogates the increase in efficacy seen with pre-administration of ex vivo activated T cells

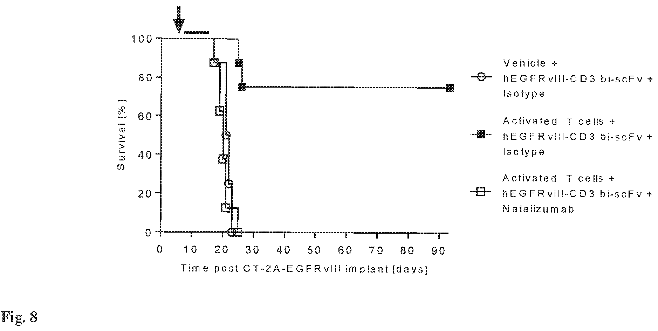

We next wondered if the increase in efficacy observed with the pre-administration of *ex vivo* modified T cells (see **Figure 5**) could be abrogated if those T cells were blocked from entering the CNS parenchyma. Natalizumab, a clinically approved drug for the treatment of multiple sclerosis, functions by binding to T cells and preventing their association with receptors involved in the process of extravasation. By blocking this interaction, T cells are unable to migrate from the systemic vasculature to the CNS parenchyma. We hypothesized that natalizumab would therefore block our *ex vivo* activated T cells from entering the CNS and therefore inhibit the process of antibody CNS hitchhiking and associated increase in efficacy. Indeed, in cohorts of mice receiving adoptive transfer of *ex vivo* activated T cells plus the extravasation blocking molecule natalizumab, efficacy was abrogated to levels observed in cohorts that did not receive adoptive transfer of activated T cells **(****Figure 8****).**

The highly invasive murine glioma CT-2A-EGFRvIII was implanted orthotopically in human CD3 transgenic mice (females, 8-10 weeks old). Where indicated (arrow), groups of mice (n=10) were given via tail vein injection either vehicle or adoptive transfer of 1x10⁷ *ex vivo* activated T cells derived from human CD3 transgenic mice. The T cells were activated with five days of *in vitro* cell culture stimulation with interleukin-2 and concanavalin A. Cohorts of mice received natalizumab or isotype control via intraperitoneal injection every other day beginning on the day of adoptive cell transfer. Natalizumab is a clinically approved drug for the treatment of multiple sclerosis that functions by blocking T cell extravasation. Where indicated (bar), cohorts of mice received daily intravenous injections with 50 µg of hEGFRvIII-CD3 bi-scFv. Pre-administration with *ex vivo* activated T cells significantly increased efficacy compared to pre-administration with vehicle, while treatment with natalizumab completely abrogated the increase in efficacy observed with pre- administration of T cells. T cell extravasation is necessary to mediate the increase in bispecific antibody efficacy induced by pre-administration with *ex vivo* activated T cells.

### EXAMPLE 9

### Pre-administration with ex vivo activated T cells significantly enhances the CNS delivery of intravenously administered EGFRvIII-CD3 binding bispecific antibody as measured with PCT/CT imaging

To demonstrate the increase in CNS penetrance of intravenously administered CD3 binding bispecific antibody molecules with pre-administration of *ex vivo* modified activated T cells we performed biodistribution experiments using both PET/CT imaging and mass spectroscopy.

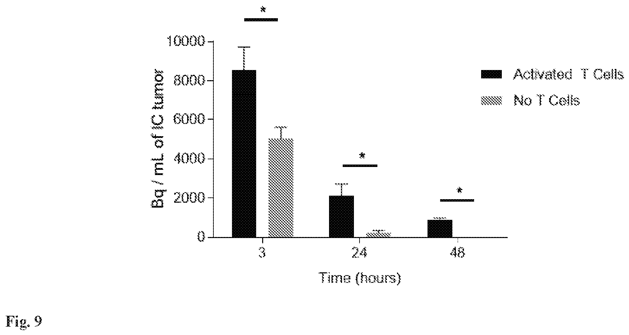

To perform biodistribution experiments using PET/CT imaging we first optimized iodine (I)-124 conjugation conditions for EGFRvIII-CD3 binding bispecific antibody. By optimizing the reaction conditions for radiolabeling the bispecific antibody, we were able to obtain a I-124 radiolabeled antibody, I-124-hEGFRvIII-CD3 bi-scFv, with equivalent binding capacity to that of the un-radiolabeled molecule. Using this radiolabeled antibody, we have found that pre-administration with *ex vivo* activated T cells significantly increases the biodistribution of hEGFRvIIII-CD3 bi-scFv to infiltrative tumors in the CNS. Cohorts of human CD3 transgenic mice bearing well-established, highly-infiltrative, orthotopic CT-2A-EGFRvIII glioma were administered *ex vivo* activated T cells or vehicle prior to administration of radiolabeled EGFRvIII-CD3 binding antibody. PET/CT imaging was performed at 3, 24, and 48 hours post intravenous administration of the radiolabeled antibody. A significant increase in radiolabeled antibody uptake was observed within the brain at each of the timepoints assessed for the cohort pre-administered with *ex vivo* activated T cells **(****Figure 9****).** These data demonstrate that *ex vivo* activated T cells can be used to significantly increase the CNS biodistribution of T cell-binding bispecific antibodies.

Groups of human CD3 transgenic mice (n=5) bearing EGFRvIII-positive CT-2A-EGFRvIII glioma (day eight tumors) were injected intravenously (IV) with 1x10⁷ *ex vivo* activated human CD3 transgenic T cells or vehicle. Four days later (day 12 tumors), 50-100 µCi of iodine-124-labeled-hEGFRvIII-CD3 bi-scFv was administered intravenously. PET/CT imaging was performed at 3, 24 and 48 hours post the IV antibody injection. A significant increase in Bq per mL of intracerebral tumor was observed at each of the time points for the cohort pre-administered *ex vivo* activated human CD3 transgenic T cells compared to the cohort that did not receive the pre-activated T cells. Increased large macromolecule biodistribution to the CNS parenchyma following intravenous administration is obtained through T cell hitchhiking.

### EXAMPLE 10

### Validation of biodistribution using mass spectrometry

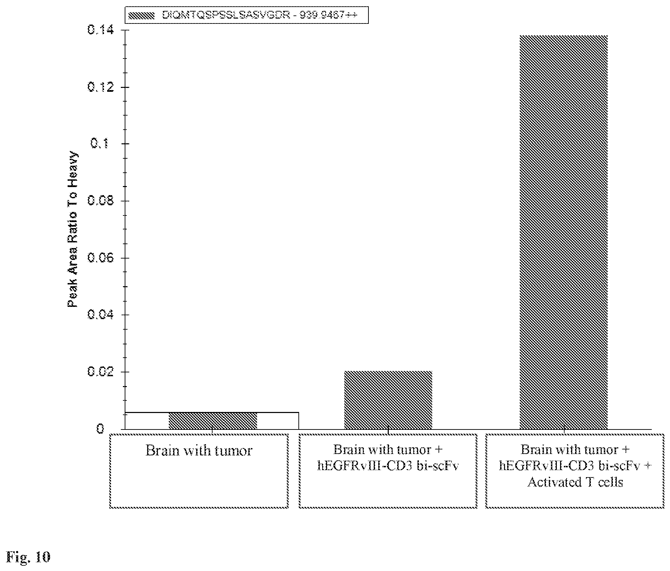

To validate further these findings in a second model, we next developed a targeted mass spectroscopy method to detect EGFRvIII-CD3 binding antibody within the brain. Using this approach, we are able to quantify the amount of unmodified, hEGFRvIII-CD3 bi-scFv within the brain parenchyma by comparison to standard curves created by adding known amounts of heavy isotope labeled hEGFRvIII-CD3 bi-scFv. Using this method, we performed experiments to compare the CNS penetration of hEGFRvIII-CD3 bi-scFv between cohorts pre-administered with *ex vivo* activated T cells or vehicle. Indeed, the amount of EGFRvIII-CD3 binding antibody that localized to the brain parenchyma was significantly higher in the activated T cell receiving cohort compared to all other groups **(****Figure 10****).** Together, these data demonstrate the CNS hitchhiking can be used to significantly increase the CNS penetrance of large, therapeutic antibodies.

Groups of human-CD3 transgenic mice were implanted with CT2A-EGFRvIII murine glioma cells orthotopically. Seven days post tumor implant, where indicated mice received 1x10⁷ *ex vivo* activated T cells or vehicle intravenously. On day 12 post tumor implant, all mice received 200 µg of CD3 binding bispecific antibody (hEGFRvIII-CD3 bi-scFv) intravenously. Three hours later, mice received intracardiac perfusions of PBS with heparin and brains were harvested. The brain concentration of hEGFRvIII-CD3 bi-scFv was measured using targeted mass spectroscopy. In brief, brains were homogenized, a heavy isotope labeled hEGFRvIII-CD3 bi-scFv was added to allow cross-sample comparisons, the homogenates were enriched for hEGFRvIII-CD3 bi-scFv using Protein-L resin, and the samples were run on a Fusion Lumos mass spectrometer. The amount of bispecific antibody that localized to the brain parenchyma was significantly higher in the activated T cell receiving cohort compared to all other groups. This occurs through a process of T cell hitchhiking, where large macromolecules bind to T cells in the periphery and are transported to the brain parenchyma as the T cell traffics to the brain.

### References

1. Hickey, W.F., Hsu, B.L. & Kimura, H. T-lymphocyte entry into the central nervous system. Journal of neuroscience research 28, 254-260 (1991).
2. Odoardi, F., et al. T cells become licensed in the lung to enter the central nervous system. Nature 488, 675-679 (2012).
3. Cole, C., et al. Tumor-targeted, systemic delivery of therapeutic viral vectors using hitchhiking on antigen- specific T cells. Nature medicine 11, 1073-1081 (2005).
4. Gedeon, P.C., et al. A Rationally Designed Fully Human EGFRvIII:CD3-Targeted Bispecific Antibody Redirects Human T Cells to Treat Patient-derived Intracerebral Malignant Glioma. Clin Cancer Res (2018).
5. Wikstrand, C.J., et al. Monoclonal antibodies against EGFRvIII are tumor specific and react with breast and lung carcinomas and malignant gliomas. Cancer Res 55, 3140-3148 (1995).
6. Humphrey, P.A., et al. Anti-synthetic peptide antibody reacting at the fusion junction of deletion-mutant epidermal growth factor receptors in human glioblastoma. Proc Natl Acad Sci U S A 87, 4207-4211 (1990).
7. Ranes, M.K., et al. N -butyldeoxynojirimycin reduces growth and ganglioside content of experimental mouse brain tumours. British journal of cancer 84, 1107-1114 (2001).
8. Ecsedy, J.A., Manfredi, M.G., Yohe, H.C. & Seyfried, T.N. Ganglioside biosynthetic gene expression in experimental mouse brain tumors. Cancer Res 57, 1580-1583 (1997).

## Claims

1. A human bispecific single chain variable fragment (scFv) that binds to EGFRvIII on the surface of tumor cells and to human CD3 on the surface of T cells, and ex vivo-activated T cells for use in treating a glioma in a brain of an immunocompetent human, wherein the T cells are a population of activated antigen-independent T cells.

2. The human bispecific single chain variable fragment (scFv) and ex vivo-activated T cells for use according to claim 1, wherein the human bispecific single chain variable fragment (scFv) and ex vivo-activated T cells are combined prior to administering.

3. The human bispecific single chain variable fragment (scFv) and ex vivo-activated T cells for use according to claim 1, wherein the human bispecific single chain variable fragment (scFv) and ex vivo-activated T cells are administered to the human within 1 week of each other.

4. The human bispecific single chain variable fragment (scFv) and ex vivo-activated T cells for use according to any one of claims 1 to 3, wherein the T cells are modified to cross the blood-brain barrier more efficiently.

5. The human bispecific single chain variable fragment (scFv) and ex vivo-activated T cells for use according to any one of claims 1 to 4, wherein the treating further comprises administering an immunomodulator to the immunocompetent human, wherein the immunomodulator is selected from the group consisting of OKT3, a chemokine, an integrin, or combinations thereof.

6. The human bispecific single chain variable fragment (scFv) and ex vivo-activated T cells for use according to any one of claims 1 to 5 wherein the scFv and the T cells are administered intravenously.

## Patentansprüche

1. Humanes bispezifisches variables Einzelkettenfragment (scFv), das an EGFRvIII auf der Oberfläche von Tumorzellen und an humanes CD3 auf der Oberfläche von T-Zellen bindet, sowie ex vivo aktivierte T-Zellen zur Verwendung bei der Behandlung eines Glioms im Gehirn eines immunkompetenten Menschen, wobei die T-Zellen eine Population aktivierter Antigen-unabhängiger T-Zellen sind.

2. Humanes bispezifisches variables Einzelkettenfragment (scFv) und ex vivo aktivierte T-Zellen zur Verwendung nach Anspruch 1, wobei das humane bispezifische variable Einzelkettenfragment (scFv) und ex vivo aktivierte T-Zellen vor der Verabreichung kombiniert werden.

3. Humanes bispezifisches variables Einzelkettenfragment (scFv) und ex vivo aktivierte T-Zellen zur Verwendung nach Anspruch 1, wobei das humane bispezifische variable Einzelkettenfragment (scFv) und ex vivo aktivierte T-Zellen innerhalb einer Woche voneinander dem Menschen verabreicht werden.

4. Humanes bispezifisches variables Einzelkettenfragment (scFv) und ex vivo aktivierte T-Zellen zur Verwendung nach einem der Ansprüche 1 bis 3, wobei die T-Zellen so modifiziert sind, dass sie die Blut-Hirn-Schranke effizienter passieren können.

5. Humanes bispezifisches variables Einzelkettenfragment (scFv) und ex vivo aktivierte T-Zellen zur Verwendung nach einem der Ansprüche 1 bis 4, wobei die Behandlung weiter die Verabreichung eines Immunmodulators an den immunkompetenten Menschen umfasst, wobei der Immunmodulator aus der Gruppe ausgewählt ist, die aus OKT3, einem Chemokin, einem Integrin oder Kombinationen davon besteht.

6. Humanes bispezifisches variables Einzelkettenfragment (scFv) und ex vivo aktivierte T-Zellen zur Verwendung nach einem der Ansprüche 1 bis 5, wobei das scFv und die T-Zellen intravenös verabreicht werden.

## Revendications

1. Fragment variable à chaîne unique bispécifique humain (scFv) qui se lie à l'EGFRvIII à la surface de cellules tumorales et au CD3 humain à la surface de lymphocytes T, et lymphocytes T activés ex vivo pour une utilisation dans le traitement d'un gliome dans le cerveau d'un être humain immunocompétent, dans lesquels les lymphocytes T sont une population de lymphocytes T activés indépendants de l'antigène.

2. Fragment variable à chaîne unique bispécifique humain (scFv) et lymphocytes T activés ex vivo pour une utilisation selon la revendication 1, dans lesquels le fragment variable à chaîne unique bispécifique humain (scFv) et les lymphocytes T activés ex vivo sont combinés avant l'administration.

3. Fragment variable à chaîne unique bispécifique humain (scFv) et lymphocytes T activés ex vivo pour une utilisation selon la revendication 1, dans lesquels le fragment variable à chaîne unique bispécifique humain (scFv) et les lymphocytes T activés ex vivo sont administrés à l'être humain à une semaine d'intervalle.

4. Fragment variable à chaîne unique bispécifique humain (scFv) et lymphocytes T activés ex vivo pour une utilisation selon l'une quelconque des revendications 1 à 3, dans lesquels les lymphocytes T sont modifiés afin de traverser plus efficacement la barrière hémato-encéphalique.

5. Fragment variable à chaîne unique bispécifique humain (scFv) et lymphocytes T activés ex vivo pour une utilisation selon l'une quelconque des revendications 1 à 4, dans lesquels le traitement comprend en outre une administration d'un immunomodulateur à l'être humain immunocompétent, dans lesquels l'immunomodulateur est sélectionné parmi le groupe consistant en l'OKT3, une chimiokine, une intégrine ou des combinaisons de celles-ci.

6. Fragment variable à chaîne unique bispécifique humain (scFv) et lymphocytes T activés ex vivo pour une utilisation selon l'une quelconque des revendications 1 à 5, dans lesquels le scFv et les lymphocytes T sont administrés par voie intraveineuse.
